# EUROPEAN PATENT APPLICATION

(11) **EP 3 093 346 A1**
(43) Date of publication of application: **16.11.2016**
(21) Application number: 15735017.4
(22) Date of filing: 06.01.2015
(51) Int. Cl.: C12P 7/22, C12Q 1/26, C12N 15/09

(54) **HYDROXYSTILBENE PRODUCTION METHOD**

(30) Priority: 10.01.2014 JP 2014003638
(71) Applicant: Morinaga & Co., Ltd., Tokyo 108-8403 (JP)
(72) Inventor: KINO, Kuniki, Tokyo 169-8050 (JP); FURUYA, Toshiki, Tokyo 169-8050 (JP)
(74) Representative: Gill Jennings & Every LLP
(86) International application number: PCT/JP2015/050177
(87) International publication number: WO 2015/105105

(57) **Abstract**

The present invention is directed to provide novel methods of producing hydroxystilbene. According to the present invention, as a method of producing a compound having the general formula (II), 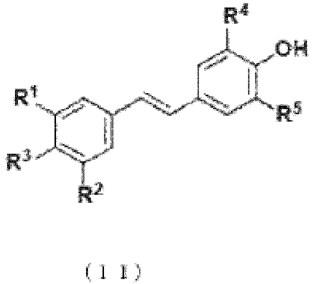 in which R¹ to R³ are each -H, -OH or -OR; R is independently selected from a C1-6 alkyl group and a glycosyl group; and one of R⁴ and R⁵ is -H and the other is -OH, 4-hydroxyphenylacetate 3-hydroxylase (HPAH) is administered to a compound having the general formula (I), 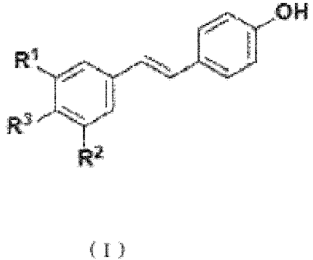 in which R¹ to R³ are each -H, -OH or -OR; and R is independently selected from a C1-6 alkyl group and a glycosyl group.

SEQUENCE LISTING

<110>  Morinaga & Co., Ltd.

<120>  Method for synthesis of hydroxystilbene

<130>  PCT2580

<160>  6

<170>  PatentIn version 3.5

<210>  1
<211>  520
<212>  PRT
<213>  Pseudomonas aeruginosa

<400>  1

Met Lys Pro Glu Asp Phe Arg Ala Ser Ala Thr Arg Pro Phe Thr Gly
1               5                   10                  15

Glu Glu Tyr Leu Ala Ser Leu Arg Asp Asp Arg Glu Ile Tyr Ile Tyr
            20                  25                  30

Gly Asp Arg Val Lys Asp Val Thr Ser His Pro Ala Phe Arg Asn Ala
        35                  40                  45

Ala Ala Ser Met Ala Arg Leu Tyr Asp Ala Leu His Asp Pro Gln Ser
    50                  55                  60

Lys Glu Lys Leu Cys Trp Glu Thr Asp Thr Gly Asn Gly Gly Tyr Thr
65                  70                  75                  80

His Lys Phe Phe Arg Tyr Ala Arg Ser Ala Asp Glu Leu Arg Gln Gln
                85                  90                  95

Arg Asp Ala Ile Ala Glu Trp Ser Arg Leu Thr Tyr Gly Trp Met Gly
            100                 105                 110

Arg Thr Pro Asp Tyr Lys Ala Ala Phe Gly Ser Ala Leu Gly Ala Asn
        115                 120                 125

Pro Gly Phe Tyr Gly Arg Phe Glu Asp Asn Ala Lys Thr Trp Tyr Lys
    130                 135                 140

Arg Ile Gln Glu Ala Cys Leu Tyr Leu Asn His Ala Ile Val Asn Pro
145                 150                 155                 160

Pro Ile Asp Arg Asp Lys Pro Val Asp Gln Val Lys Asp Val Phe Ile
                165                 170                 175

Ser Val Asp Glu Glu Val Asp Gly Gly Ile Val Val Ser Gly Ala Lys
        180                 185                 190

Val Val Ala Thr Asn Ser Ala Leu Thr His Tyr Asn Phe Val Gly Gln
    195                 200                 205

Gly Ser Ala Gln Leu Leu Gly Asp Asn Thr Asp Phe Ala Leu Met Phe
    210                 215                 220

Ile Ala Pro Met Asn Thr Pro Gly Met Lys Leu Ile Cys Arg Pro Ser
225                 230                 235                 240

Tyr Glu Leu Val Ala Gly Ile Ala Gly Ser Pro Phe Asp Tyr Pro Leu
                245                 250                 255

Ser Ser Arg Phe Asp Glu Asn Asp Ala Ile Leu Val Met Asp Lys Val
            260                 265                 270

Phe Ile Pro Trp Glu Asn Val Leu Ile Tyr Arg Asp Phe Glu Arg Cys
        275                 280                 285

Lys Gln Trp Phe Pro Gln Gly Gly Phe Gly Arg Leu Phe Pro Met Gln
    290                 295                 300

Gly Cys Thr Arg Leu Ala Val Lys Leu Asp Phe Ile Thr Gly Ala Leu
305                 310                 315                 320

Tyr Lys Ala Leu Gln Cys Thr Gly Ser Leu Glu Phe Arg Gly Val Gln
                325                 330                 335

Ala Gln Val Gly Glu Val Val Ala Trp Arg Asn Leu Phe Trp Ser Leu
            340                 345                 350

Thr Asp Ala Met Tyr Gly Asn Ala Ser Glu Trp His Gly Gly Ala Phe
        355                 360                 365

Leu Pro Ser Ala Glu Ala Leu Gln Ala Tyr Arg Val Leu Ala Pro Gln
    370                 375                 380

Ala Tyr Pro Glu Ile Lys Lys Thr Ile Glu Gln Val Val Ala Ser Gly
385                 390                 395                 400

Leu Ile Tyr Leu Pro Ser Gly Val Arg Asp Leu His Asn Pro Gln Leu
                405                 410                 415

Asp Lys Tyr Leu Ser Thr Tyr Cys Arg Gly Ser Gly Gly Met Gly His
            420                 425                 430

Arg Glu Arg Ile Lys Ile Leu Lys Leu Leu Trp Asp Ala Ile Gly Ser
        435                 440                 445

Glu Phe Gly Gly Arg His Glu Leu Tyr Glu Ile Asn Tyr Ala Gly Ser
    450                 455                 460

Gln Asp Glu Ile Arg Met Gln Ala Leu Arg Gln Ala Ile Gly Ser Gly
465                 470                 475                 480

Ala Met Lys Gly Met Leu Gly Met Val Glu Gln Cys Met Gly Asp Tyr
                485                 490                 495

Asp Glu Asn Gly Trp Thr Val Pro His Leu His Asn Pro Asp Asp Ile
            500                 505                 510

Asn Val Leu Asp Arg Ile Arg Gln
        515                 520

<210>  2
<211>  170
<212>  PRT
<213>  Pseudomonas aeruginosa

<400>  2

Met Ser Gln Leu Glu Pro Arg Gln Gln Ala Phe Arg Asn Ala Met Ala
1               5                   10                  15

His Leu Ser Ala Ala Val Asn Val Ile Thr Ser Asn Gly Pro Ala Gly
            20                  25                  30

Arg Cys Gly Ile Thr Ala Thr Ala Val Cys Ser Val Thr Asp Ser Pro
        35                  40                  45

Pro Thr Leu Met Leu Cys Ile Asn Arg Asn Ser Glu Met Asn Thr Val
    50                  55                  60

Phe Lys Ala Asn Gly Arg Leu Cys Val Asn Val Leu Ser Gly Glu His
65                  70                  75                  80

Glu Glu Val Ala Arg His Phe Ala Gly Met Thr Glu Val Pro Met Glu
                85                  90                  95

Arg Arg Phe Ala Leu His Asp Trp Arg Glu Gly Leu Ala Gly Leu Pro
            100                 105                 110

Val Leu His Gly Ala Leu Ala Asn Leu Gln Gly Arg Ile Ala Glu Val
        115                 120                 125

Gln Glu Ile Gly Thr His Ser Val Leu Leu Leu Glu Leu Glu Asp Ile
    130                 135                 140

Gln Val Leu Glu Gln Gly Asp Gly Leu Val Tyr Phe Ser Arg Ser Phe
145                 150                 155                 160

His Arg Leu Gln Cys Pro Arg Arg Ala Ala
                165                 170

<210>  3
<211>  30
<212>  DNA
<213>  Pseudomonas aeruginosa

<400>  3
ttctcatgaa acccgaagat ttccgtgcct                                        30

<210>  4
<211>  30
<212>  DNA
<213>  Pseudomonas aeruginosa

<400>  4
cgcggatcct cattggcgga tgcgatcgag                                        30

<210>  5
<211>  30
<212>  DNA
<213>  Pseudomonas aeruginosa

<400>  5
ttccatatgt cccagctcga acccaggcag                                        30

<210>  6
<211>  30
<212>  DNA
<213>  Pseudomonas aeruginosa

<400>  6
ttcggtacct caggccgccc gccgggggca                                        30

## Description

### Technical field

The present invention relates to methods of producing hydroxystilbene.

### Background art

Piceatannol (i.e., 3',4',3,5-tetrahydroxy-trans-stilbene or 3,3',4',5-tetrahydroxy-trans-stilbene) having the structure of resveratrol which has been hydroxylated at the 3'-position is a notable compound that has been shown to exhibit various physiological activities such as anti-oxidative activity and anti-cancer activity (Mutat Res-Fund Mol M vol. 750, p. 60-82 (2012)). Focusing on the various physiological activities of piceatannol, extracts of the passion fruit seeds containing the compound have been investigated for the purpose of healthcare and medical applications (JP-A-2010-030911, JP-A-2013-151457, and JP-A-2013-151458).

Although piceatannol is contained in plants such as passion fruits and grapes, its extraction from the plants is not efficient at all because the content is small (WO2010/113315). Piceatannol can also be obtained by organic synthesis but many complicated reaction procedures are required.

It has recently been disclosed that 4-hydroxyphenylacetate 3-hydroxylases (HPAHs; EC 1.14.13.3) (synonym: p-hydroxyphenylacetate 3-hydroxylase, 4-hydroxyphenylacetate 3-monooxygenase, p-hydroxyphenylacetate hydroxylase, 4-HPA 3-hydroxylase) from *Escherichia coli* are capable of converting resveratrol into piceatannol (Curr Opin Biotech vol. 26 p. 71-78 (2014)). Yields are, however, still unsatisfactory.

### Summary of the invention

An object of the present invention is to provide novel methods of producing hydroxystilbene.

### Means to solve the problems

4-hydroxyphenylacetate 3-hydroxylases (HPAHs; EC 1.14.13.3) (synonyms: p-hydroxyphenylacetate 3-hydroxylase, 4-hydroxyphenylacetate 3-monooxygenase, p-hydroxyphenylacetate hydroxylase, 4-HPA 3-hydroxylase) are known to be two-component FAD-dependent monooxygenases and be capable of synthesizing ortho-diphenol compounds from monophenol compounds using flavin as a coenzyme.

The present inventors searched for an effective method of producing piceatannol and then found that HPAHs are capable of efficiently converting resveratrol into piceatannol. The present invention was thus completed.

An aspect of the present invention is a method of producing a compound having the general formula (II): 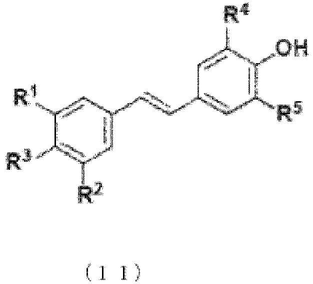 in which R¹ to R³ are each -H, -OH or -OR; R is independently selected from a C1-6 alkyl group and a glycosyl group; and one of R⁴ and R⁵ is -H and the other is -OH, including the step of administering 4-hydroxyphenylacetate 3-hydroxylase (HPAH) to a compound having the general formula (I): 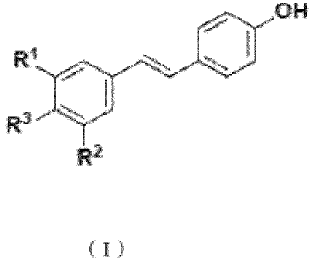 in which R¹ to R³ are each -H, -OH or -OR; and R is independently selected from a C1-6 alkyl group and a glycosyl group.

Another aspect of the present invention is a method of producing a compound having the general formula (III): 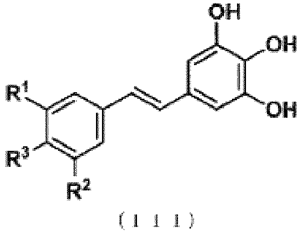 in which R¹ to R³ are each -H, -OH or -OR; and R is independently selected from a C1-6 alkyl group and a glycosyl group, including the step of administering 4-hydroxyphenylacetate 3-hydroxylase (HPAH) to a compound having the general formula (I): 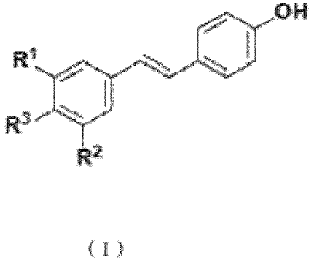 in which R¹ to R³ are each -H, -OH or -OR; and R is independently selected from a C1-6 alkyl group and a glycosyl group, and/or a compound having the general formula (II): 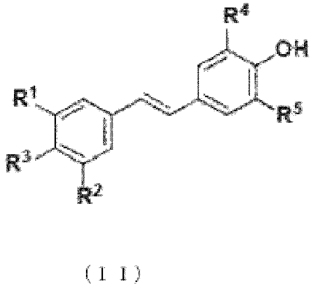 in which R¹ to R³ are each -H, -OH or -OR; R is independently selected from a C1-6 alkyl group and a glycosyl group; and one of R⁴ and R⁵ is -H and the other is -OH.

In either method described above, the compound having the general formula (II) may be piceatannol. The HPAH may be a recombinant protein or a natural protein.

In either method described above, a purified HPAH recombinant protein, a purified HPAH natural protein, a bacterium expressing an endogenous HPAH or an extract from the bacterium, or a microorganism expressing an exogenous HPAH or an extract from such microorganism may be administered to the compound that is a substrate. The bacterium expressing an endogenous HPAH may be *Pseudomonas aeruginosa.*

A further aspect of the present invention is a method of measuring an enzymatic activity to convert a compound having the general formula (I): 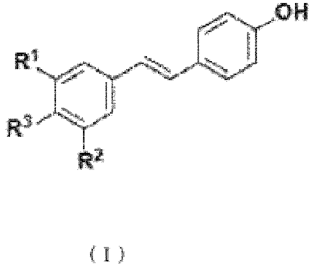 in which R¹ to R³ are each -H, -OH or -OR; and R is independently selected from a C1-6 alkyl group and a glycosyl group, into a compound having the general formula (II): 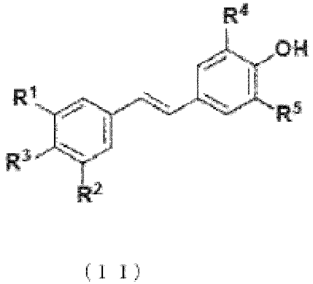 in which R¹ to R³ are each -H, -OH or -OR; R is independently selected from a C1-6 alkyl group and a glycosyl group; and one of R⁴ and R⁵ is -H and the other is -OH, including the step of administering a 4-hydroxyphenylacetate 3-hydroxylase (HPAH) analog to the compound having the general formula (I) to detect the compound having the general formula (II), the analog being a protein having an amino acid sequence which is at least 50% homologous to HpaB or HpaC derived from a *Pseudomonas aeruginosa* PAO1 strain.

A yet another aspect of the present invention is a method of screening 4-hydroxyphenylacetate 3-hydroxylase (HPAH) analogs for an enzyme capable of converting a compound having the general formula (I) : 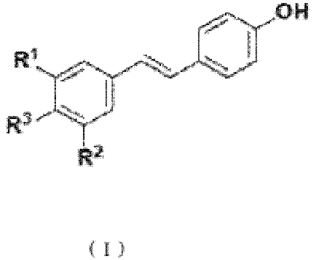 in which R¹ to R³ are each -H, -OH or -OR; and R is independently selected from a C1-6 alkyl group and a glycosyl group, the analog being a protein having an amino acid sequence which is at least 50% homologous to HpaB or HpaC derived from a *Pseudomonas aeruginosa* PAO1 strain, into a compound having the general formula (II): 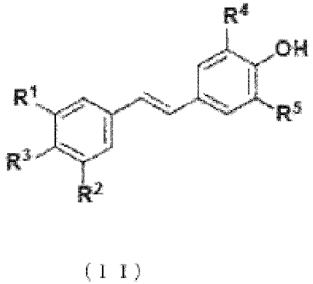 in which R¹ to R³ are each -H, -OH or -OR; R is independently selected from a C1-6 alkyl group and a glycosyl group; and one of R⁴ and R⁵ is -H and the other is -OH, including the steps of administering the 4-hydroxyphenylacetate 3-hydroxylase (HPAH) analogs to the compound having the general formula (I) to detect the compound having the general formula (II); and selecting an HPAH analog with which the compound having the general formula (II) is detected.

A still another aspect of the present invention is a method of measuring an enzymatic activity to convert a hydroxyphenyl compound as a substrate into a dihydroxyphenyl compound including the step of administering 4-hydroxyphenylacetate 3-hydroxylase (HPAH) to the hydroxyphenyl compound to detect the dihydroxyphenyl compound, the hydroxyphenyl compound being a compound having the general formula (I) : 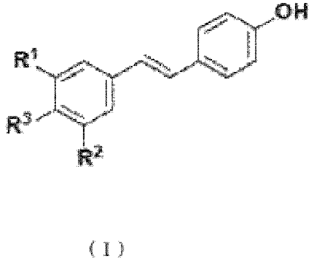 in which R¹ to R³ are each -H, -OH or -OR; and R is independently selected from a C1-6 alkyl group and a glycosyl group.

Another aspect of the present invention is a method of screening hydroxyphenyl compounds as a substrate of 4-hydroxyphenylacetate 3-hydroxylase (HPAH) that catalyzes conversion of a hydroxyphenyl compound into a dihydroxyphenyl compound including the steps of administering the 4-hydroxyphenylacetate 3-hydroxylase (HPAH) to the hydroxyphenyl compounds to detect the dihydroxyphenyl compound; and selecting the substrate of HPAH with which the dihydroxyphenyl compound is detected, the hydroxyphenyl compound being a compound having the general formula (I): 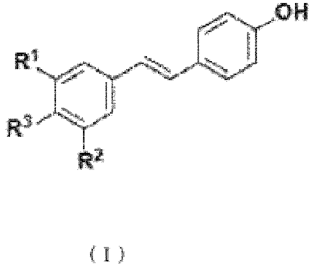 in which R¹ to R³ are each -H, -OH or -OR; and R is independently selected from a C1-6 alkyl group and a glycosyl group.

### Brief description of the drawings

Fig. 1 is a schematic diagram illustrating (A) the mechanism of conversion and (B) the mechanism of conversion in the bacterial reaction system, through which HPAH converts a compound.
Fig. 2 is amino acid sequences of HpaBC derived from the *Pseudomonas aeruginosa* PAO1 strain.
Fig. 3 shows a result of HPLC analysis for the conversion of resveratrol by HpaBC-expressing recombinant *Escherichia coli* in an example of the present invention.
Fig. 4 is a graph showing a result of piceatannol synthesis by HpaBC-expressing recombinant *Escherichia coli* on a flask scale in an example of the present invention.

### Embodiments for carrying out the invention

Unless otherwise noted in embodiments and examples, all procedures used are according to standard protocols such as J. Sambrook, E. F. Fritsch & T. Maniatis (Ed.), Molecular cloning, a laboratory manual (3rd edition), Cold Spring Harbor Press, Cold Spring Harbor, New York (2001); and F. M. Ausubel, R. Brent, R. E. Kingston, D. D. Moore, J.G. Seidman, J. A. Smith, K. Struhl (Ed.), Current Protocols in Molecular Biology, John Wiley & Sons Ltd., with or without modifications or changes. In addition, commercial reagent kits or measurement instruments are used as described in protocols attached thereto, unless otherwise noted.

The objects, features, advantages, and ideas of the present invention are apparent to those skilled in the art from the description of this specification. Those skilled in the art can easily reproduce the present invention from the description herein. The embodiments and specific examples described below represent preferable aspects of the present invention, which are given for the purpose of illustration or explanation. The present invention is not limited thereto. It is obvious to those skilled in the art that various changes and modifications may be made according to the description of the present specification within the spirit and scope of the present invention disclosed herein.

### == Method of converting compounds ==

According to an embodiment of the present invention, it is possible to synthesize the compound having the general formula (II) (hereinafter, referred to as compound (II)) by administering the enzyme HPAH to the compound having the general formula (I) (hereinafter, referred to as compound (I)). Furthermore, this reaction can be used to measure the activity of the HPAH enzyme that catalyzes conversion of the compound (I) into the compound (II). 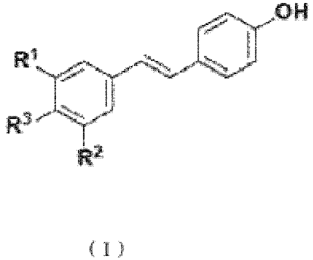 in which R¹ to R³ are each -H, -OH or -OR; and R is independently selected from a C1-6 alkyl group and a glycosyl group; and 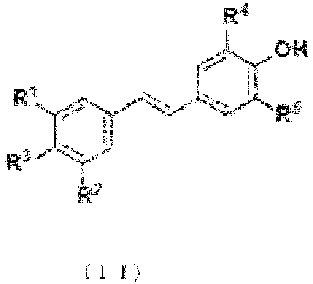 in which R¹ to R³ are each -H, -OH or -OR; R is independently selected from a C1-6 alkyl group and a glycosyl group; and one of R⁴ and R⁵ is -H and the other is -OH.

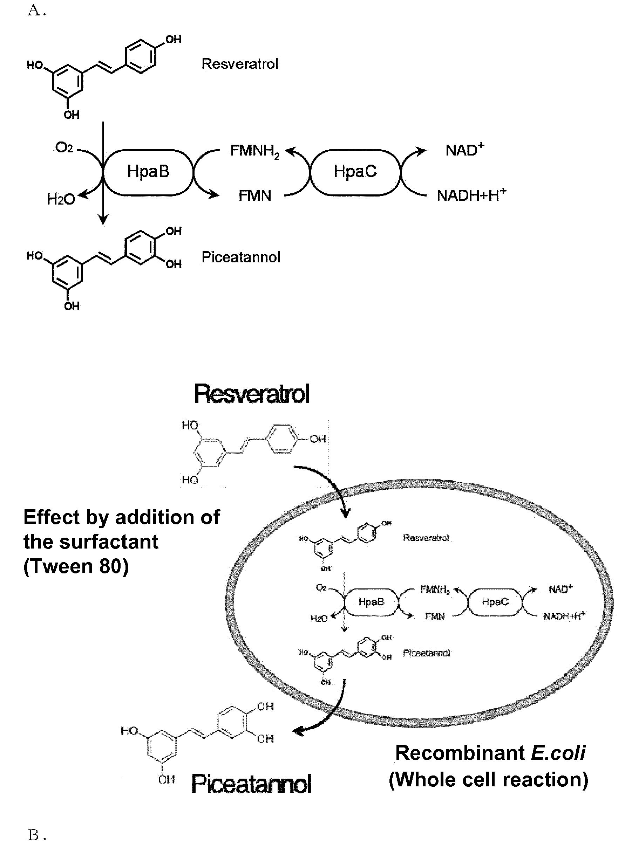

It is preferable that R³ is -H. Furthermore, it is preferable that both R¹ and R² are -OH or -OR, and R is independently selected from a C1-6 alkyl group and a glycosyl group. It is most preferable that both R¹ and R² are -OH; in this case, the compound (I) is resveratrol and the compound (II) is piceatannol. A mechanism of conversion activity of the enzyme during the conversion is shown in Fig. 1. 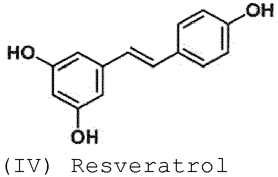 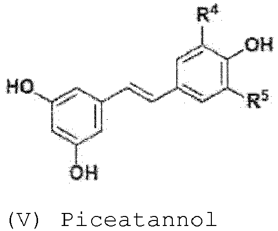 in which one of R⁴ and R⁵ is -H and the other is -OH.

Moreover, by administering HPAH for a long time, a compound having the general formula (III) (hereinafter, referred to as a compound (III)) can be synthesized from the compound (I) or the compound (II) : 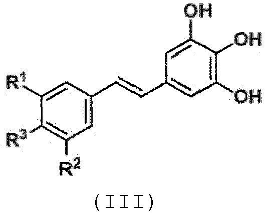 in which R¹ to R³ are each -H, -OH or -OR; and R is independently selected from a C1-6 alkyl group and a glycosyl group.

The two-component flavin-dependent oxygenase HPAH to be administered consists of HpaB which is a subunit of monooxygenase and HpaC which is a subunit of oxidoreductase. The source of HpaB and HpaC is not specifically limited and examples include bacteria classified into genera such as *Pseudomonas, Escherichia, Enterobacter, Krebsiella, Salmonella, Serratia, Shigella, Yersinia, Rhizobium, Bradyrhizobium, Mesorhizobium, Sinorhizobium, Bacillus, Geobacillus, Saccharothrix, Streptomyces, Rhodococcus, Gordonia, Mycobacterium, Thermus, Acinetbacter, Halomonas,* and *Micrococcus.* A pseudomonad *such as Pseudomonas putida, Pseudomonas aeruginosa,* and *Pseudomonas ovalis* is preferable, and *Pseudomonas aeruginosa* or *Escherichia coli* is particularly preferable. For *Pseudomonas aeruginosa,* a PAO1 strain is more preferable. For *Escherichia coli,* a W strain or a B strain is preferable. It should be noted that HpaB and HpaC herein include all homologs and mutants which are capable of functioning as a two-component flavin-dependent oxygenase when interacted with each other, unless the enzyme is specified based on a species. Each homolog or mutant may have a natural sequence or an artificial sequence. HpaB and HpaC may be derived from the same species or different species. At least one of them may have an artificial sequence. The homolog or mutant is a protein having an amino acid sequence which is at least 70%, preferably at least 80%, more preferably at least 90%, and yet more preferably at least 95% homologous to HpaB or HpaC of *Pseudomonas aeruginosa.* The method of measuring homology is not particularly limited and examples include BLAST, FASTA, and PSI-BLAST.

The type of HpaB and HpaC is not specifically limited. They may independently be a recombinant protein or a natural protein.

As way of example where HPAH is a recombinant protein, when the hpaB gene that encodes HpaB and the hpaC gene that encodes HpaC, which make up HPAH, are both introduced into a host microorganism and allowed to be expressed therein, HPAH is reconstructed in the recombinant microorganism. Since the compound (I) penetrates through the cell membrane, addition of the compound (I) to the medium in which the recombinant microorganism has been cultured results in synthesis of the compound (II) and/or the compound (III) in the cells. Accordingly, the recombinant microorganism itself can be used for the conversion of the compound. The host microorganism is not specifically limited and examples include bacteria and yeasts. The host microorganism is preferably *Escherichia coli.* When the enzyme is a natural protein, bacteria expressing an endogenous HPAH can be used instead of the recombinant microorganism as described above. Fig. 1(B) shows a mechanism of enzymatic conversion activity in the bacterial reaction system.

When a microorganism is used for the conversion of the compounds, compound-conversion efficiency increases when a surfactant is added to a medium. The type of the surfactant to be used is not specifically limited and examples include SDS, Triton X-100, and Tween 80. The concentration of the surfactant is not also specifically limited and those skilled in the art can choose an appropriate concentration. The concentration is preferably from about 0.1% to about 10%, both inclusive, more preferably from about 0.5% to about 2%, both inclusive, and most preferably about 1%.

For the conversion of the compounds, an extract of a bacterium expressing an endogenous HPAH or a recombinant microorganism, or HPAH purified from a bacterium expressing an endogenous HPAH or a recombinant microorganism may be used rather than using a microorganism. The purification process is not specifically limited and may be crude purification or high-purity purification. When an extract or a purified HPAH is used, the compound can be converted in vitro by providing a reconstruction system with the addition of, for example, FAD or NAD (see, e.g., Biochemistry vol. 49, p. 372-385 (2010)).

### == Method of measuring activity of conversion of a compound ==

In one embodiment of the present invention, the enzymatic activity of conversion of the compound (I) to the compound (II) can be measured by administering a 4-hydroxyphenylacetate 3-hydroxylase (HPAH) analog to the compound (I) to detect the compound (II). The HPAH analog herein is a homolog of HpaB (SEQ ID NO.: 1) or HpaC (SEQ ID NO.: 2) derived from the *Pseudomonas aeruginosa* PAO1 strain and is a protein having an amino acid sequence which is at least 50%, 60%, 70%, 80%, or 90% homologous to HpaB or HpaC. It is thus possible to determine whether a given HPAH analog has the activity of conversion of the compound. The method of measuring the homology is not specifically limited and examples include BLAST, FASTA, and PSI-BLAST.

### == Method of screening enzymes for those having activity of conversion of a compound ==

In one embodiment of the present invention, it is possible to screen 4-hydroxyphenylacetate 3-hydroxylase (HPAH) analogs for an enzyme converting the compound (I) to the compound (II) by administering a plurality of HPAH analogs to the compound (I), detecting the compound (II), and selecting the HPAH analog (s) for which the conversion has been detected. The HPAH analogs herein are proteins having an amino acid sequence which is at least 50%, 60%, 70%, 80%, or 90% homologous to HpaB or HpaC derived from the *Pseudomonas aeruginosa* PAO1 strain. It is thus possible to determine whether a given HPAH analog has the activity of conversion of the compound. The method of measuring the homology is not specifically limited and examples include BLAST, FASTA, and PSI-BLAST.

### Examples

### (1) Construction of recombinant Escherichia coli expressing two-component flavin-dependent oxidase HpaBC

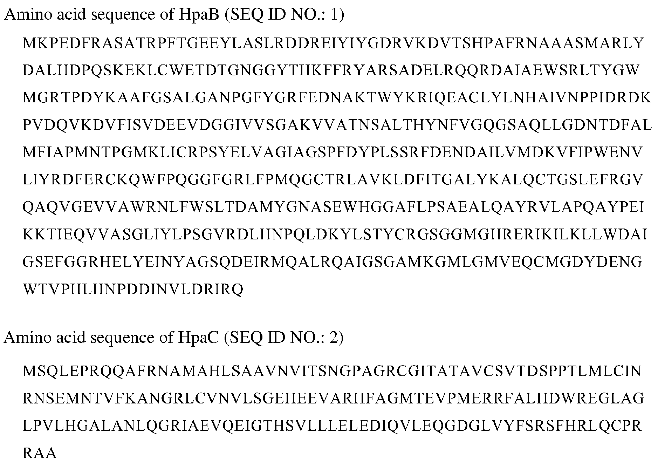

A gene that encodes the two-component flavin-dependent oxidase HpaBC of which amino acid sequence is shown in Fig. 2 was inserted into the pETDuet-1 vector (Novagen) and the recombinant vector was introduced into the *Escherichia coli* BL21 Star (DE3) strain (Invitrogen).

More specifically, the hpaB gene (ORF no. PA4091) that encodes a monooxygenase component and the hpaC gene(ORF no. PA4092) that encodes an oxide reductase component were amplified with PCR using genome of the *Pseudomonas aeruginosa* PAO1 strain (ATCC 47085D-5, GenBank Accession no. NC#002516) as template. Sense primer hpaB-s and antisense primer hpaB-as used for the amplification of the hpaB gene as well as sense primer hpaC-s and antisense primer hpaC-as used for the amplification of the hpaC gene are given below.
hpaB-s: TTCTCATGAAACCCGAAGATTTCCGTGCCT (SEQ ID NO.: 3)
hpaB-as: CGCGGATCCTCATTGGCGGATGCGATCGAG (SEQ ID NO.: 4)
hpaC-s: TTCCATATGTCCCAGCTCGAACCCAGGCAG (SEQ ID NO.: 5)
hpaC-as: TTCGGTACCTCAGGCCGCCCGCCGGGGGCA (SEQ ID NO.: 6)

After the amplification using PCR, both ends were digested with restriction enzymes BspHI and BamHI for the hpaB gene and NdeI and KpnI for the hpaC gene and then the respective DNAs were inserted into 1 and 2 of the multi-cloning site of pETDuet-1. The constructed plasmid (pETDhpaBC) was introduced into the E. *coli* BL21 Star (DE3) strain using electroporation.

### (2) Identification of a conversion product of resveratrol, produced by a HpaBC-expressing recombinant Escherichia coli

The constructed recombinant *Escherichia coli* was inoculated into LB medium (tryptone 1%, yeast extract 0.5%, NaCl 1%, pH 7.0) containing 100 µg/ml of ampicillin and incubated at 25°C. After 12 hours, 1 mM of isopropyl-β-D-thiogalactopyranoside was added and incubated for additional 12 hours to induce expression of the hpaBC gene. The bacteria were harvested, washed with 50mM of potassium phosphate buffer (pH 7.5) containing 10% (v/v) of glycerol, and suspended in the same buffer. The suspension was used for the reaction with resveratrol; specifically, 100 µl of a reaction mixture consisting of 8 g/l (dry cell weight) of HpaBC-expressing recombinant *Escherichia coli,* 10 mM of resveratrol, 4% (v/v) of dimethyl sulfoxide, 10% (v/v) of glycerol, and 50 mM of potassium phosphate buffer (pH 7.5) was prepared and shaken at 30°C for 24 hours.

After the reaction, 10 µl of 5N HCl, 400 µl of H₂O, and 500 µl of methanol were added to prepare a sample for HPLC analysis. The HPLC analysis was performed using a system (1100 series) manufactured by Agilent Technologies and a column (XTerra MS C18 IS, length 4.6 x 20 mm, particle diameter: 3.5 µm) manufactured by Waters Corporation. For the developing solvent, 10 mM of acetonitrile/methanol/potassium phosphate buffer (pH 2.7) = 2.5/2.5/95 (solvent A) and acetonitrile (solvent B) were used. The sample was eluted at a flow rate of 1 ml/min starting at 0% solvent B over 0-3 min, and then increasing the solvent B with a linear gradient to 70% over 3-12 min.

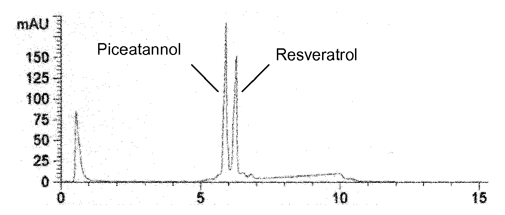

As a result, a new peak (5.9 min) was detected in addition to the peak of the resveratrol (6.3 min) (Fig. 3). The retention time of the peak was the same as the standard piceatannol, and its PDA spectrum was also identical with that of piceatannol.

Furthermore, NMR and LC-MS analyses were carried out and it was shown that the NMR spectrum and mass of the compound corresponding to the new peak were the same as those of piceatannol. Thus, the conversion product of resveratrol was identified as piceatannol. The results of the NMR and LC-MS analyses were given below.

¹H NMR (600 MHz, [D₆]DMSO): δ = 6.13 (dd, J = 1.8, 1.8 Hz, 1H), 6.39 (d, J = 1.8 Hz, 2H), 6.73 (d, J = 17.6 Hz, 1H), 6.73 (d, J = 7.8 Hz, 1H), 6.84 (dd, J = 7.8, 1.8 Hz, 1H), 6.86 (d, J = 17.6 Hz, 1H), 6. 98 (d, J = 1.8 Hz, 1H) ; ¹³C NMR (600 MHz, [D₆]DMSO) δ = 102.2, 104.8, 113,7, 116,2, 119.0, 126.0, 128.7, 129.1, 139.7, 145.8, 145.9, 158.9; MS (ESI) (m/z) : calculated for C₁₄H₁₁O₄ [M-H]⁻, 243.1; found, 243.2.

As apparent from the above, resveratrol can be converted into piceatannol by administering the HpaBC-expressing recombinant *Escherichia coli* to resveratrol.

When the reaction was continued for a longer time, a new peak (5.4 min) was detected in addition to the peak of piceatannol in the HPLC analysis. LC-MS analysis was carried out and it was shown that a compound corresponding to this peak had [M-H]⁻ of 259.2 and was identified as a compound in which two oxygen atoms had been added to resveratrol. This indicated that resveratrol was converted to the following compound through piceatannol.

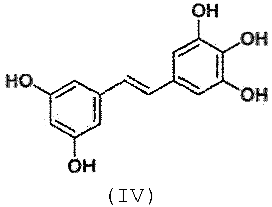

### (3) Investigation of conditions for resveratrol conversion

Effects of the concentrations of the substrate, the bacteria, and the surfactant on the resveratrol conversion activity were examined (Table 1). First, the bacterial cells (8 g/l in dry cell weight) were administered to 10 mM of the substrate under the same conditions as (2) to produce 4.3 mM of piceatannol in 24 hours (Entry 1). When the concentrations of the substrate and the bacteria were changed (Entries 2-5), the amount of the product piceatannol was increased to 8.8 mM (Entry 5). Furthermore, when the surfactant was added to the reaction mixture (Entries 6-9), the amount of the product was significantly increased at the concentration of 1% (v/v). In particular, in the presence of 1% (v/v) of Tween 80, 25 mM (6.1 g/l) of piceatannol was produced from 30 mM of resveratrol at a molar conversion yield of 83% (Entry 7). This is more than six times higher than the yield obtained when the HpaBC derived from *Escherichia coli* was used (Curr Opin Biotech vol. 26, p. 71-78 (2014)).

**[Table 1]**

| Investigation of resveratrol conversion conditions using HpaBC-expressing *Escherichia coli* | | | | | |
|---|---|---|---|---|---|
| Entry | Substrate (mM) | Cells (g/l)^{a} | Detergent (% v/v) | Product (mM) | Conversion (%)^{b} |
| 1 | 10 | 8 | 0 | 4.3 | 43 |
| 2 | 10 | 16 | 0 | 6.2 | 62 |
| 3 | 20 | 16 | 0 | 7.2 | 36 |
| 4 | 20 | 32 | 0 | 8.1 | 41 |
| 5 | 30 | 32 | 0 | 8.8 | 29 |
| 6 | 30 | 32 | 0.1 (Tween 80) | 13 | 43 |
| 7 | 30 | 32 | 1 (Tween 80) | 25 | 83 |
| 8 | 30 | 32 | 0.1 (TritonX-100) | 9.9 | 33 |
| 9 | 30 | 32 | 1 (TritonX-100) | 22 | 73 |

| | | | | | |
|---|---|---|---|---|---|
| Dry well weight/liter ^{b} Conversion (%) = Product (mM)/Substrate (mM) x 100 | | | | | |

### (4) Synthesis of piceatannol on a flask scale

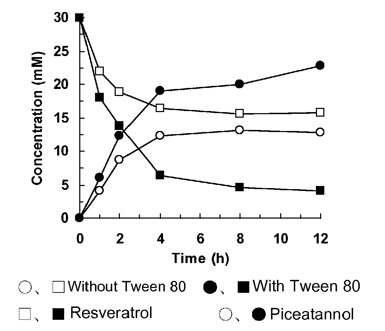

Piceatannol was synthesized on a flask scale under the optimum reaction condition determined in (3); more specifically, 20 ml of reaction mixture consisting of 32 g/l (dry cell weight) of HpaBC-expressing recombinant *Escherichia coli* cells, 30 mM of resveratrol, 4% (v/v) of dimethyl sulfoxide, 10% (v/v) of glycerol, and 50 mM of potassium phosphate buffer (pH 7.5) was prepared and shaken at 30°C. As a result, 13 mM (2.9 g/l) of piceatannol was produced in 12 hours without addition of the surfactant Tween 80 whereas 23 mM (5.2 g/l) of piceatannol was produced in the presence of 1% (v/v) of Tween 80 (Fig. 4). As apparent from the above, piceatannol could be also synthesized in an efficient manner on a flask scale.

### Industrial applicability

The present invention makes it possible to provide novel methods of producing hydroxystilbene.

## Claims

1. A method of producing a compound having the general formula (II): 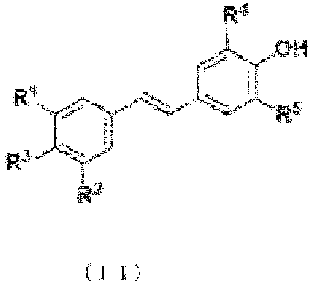 wherein
R¹ to R³ are each -H, -OH or -OR;
R is independently selected from a C1-6 alkyl group and a glycosyl group; and
one of R⁴ and R⁵ is -H and the other is -OH, comprising the step of administering 4-hydroxyphenylacetate 3-hydroxylase (HPAH) to a compound having the general formula (I): 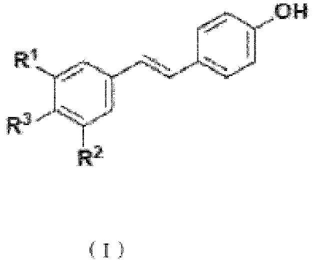 wherein
R¹ to R³ are each -H, -OH or -OR; and
R is independently selected from a C1-6 alkyl group and a glycosyl group.

2. A method of producing a compound having the general formula (III) : 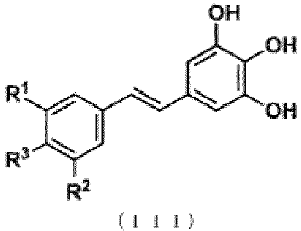 wherein
R¹ to R³ are each -H, -OH or -OR; and
R is independently selected from a C1-6 alkyl group and a glycosyl group,
comprising the step of administering 4-hydroxyphenylacetate 3-hydroxylase (HPAH) to a compound having the general formula (I): 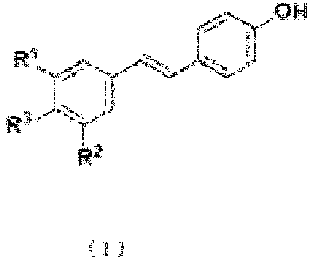 wherein
R¹ to R³ are each -H, -OH or -OR; and
R is independently selected from a C1-6 alkyl group and a glycosyl group,
and/or a compound having the general formula (II): 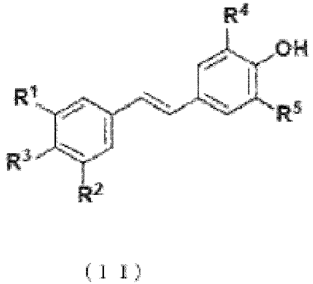 wherein
R¹ to R³ are each -H, -OH or -OR;
R is independently selected from a C1-6 alkyl group and a glycosyl group; and
one of R⁴ and R⁵ is -H and the other is -OH.

3. The method according to Claim 1 or 2, wherein R³ is -H.

4. The method according to Claim 3, wherein the compound having the general formula (II) is piceatannol.

5. The method according to any one of Claims 1 to 4, wherein the HPAH is a recombinant protein or a natural protein.

6. The method according to any one of Claims 1 to 5, wherein a purified HPAH recombinant protein, a purified HPAH natural protein, an extract from a bacterium expressing an endogenous HPAH, or an extract from a microorganism expressing an exogenous HPAH is administered to the compound that is a substrate.

7. The method according to any one of Claims 1 to 5, wherein a bacterium expressing an endogenous HPAH or a microorganism expressing an exogenous HPAH is administered to the compound that is a substrate.

8. The method according to Claim 6, wherein the bacterium expressing an endogenous HPAH is *Pseudomonas aeruginosa.*

9. The method according to Claim 7 or 8, wherein the bacterium expressing an endogenous HPAH or the microorganism expressing an exogenous HPAH is administered to the compound in the presence of a surfactant.

10. The method according to Claim 11, wherein the compound having the general formula (II) is piceatannol.

11. A method of measuring an enzymatic activity to convert a compound having the general formula (I): 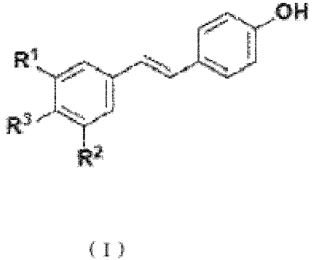 wherein
R¹ to R³ are each -H, -OH or -OR; and
R is independently selected from a C1-6 alkyl group and a glycosyl group,
into a compound having the general formula (II): 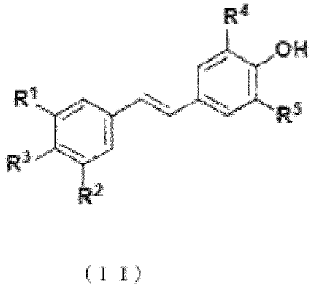 wherein
R¹ to R³ are each -H, -OH or -OR;
R is independently selected from a C1-6 alkyl group and a glycosyl group; and
one of R⁴ and R⁵ is -H and the other is -OH,
comprising the step of:
administering a 4-hydroxyphenylacetate 3-hydroxylase (HPAH) analog to the compound having the general formula (I) to detect the compound having the general formula (II),
the analog being a protein having an amino acid sequence which is at least 50% homologous to HpaB or HpaC derived from a *Pseudomonas aeruginosa* PAO1 strain.

12. A method of screening 4-hydroxyphenylacetate 3-hydroxylase (HPAH) analogs for an enzyme converting a compound having the general formula (I): 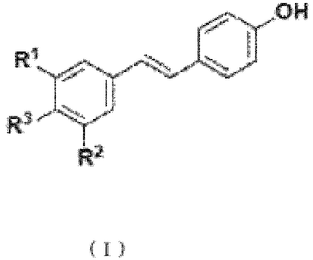 wherein
R¹ to R³ are each -H, -OH or -OR; and
R is independently selected from a C1-6 alkyl group and a glycosyl group,
the analog being a protein having an amino acid sequence which is at least 50% homologous to HpaB or HpaC derived from a *Pseudomonas aeruginosa* PAO1 strain,
into a compound having the general formula (II): 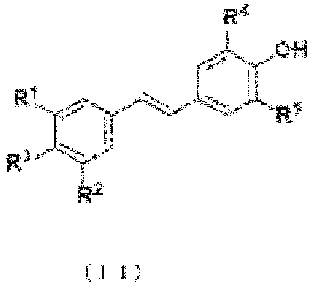 wherein
R¹ to R³ are each -H, -OH or -OR;
R is independently selected from a C1-6 alkyl group and a glycosyl group; and
one of R⁴ and R⁵ is -H and the other is -OH,
comprising the steps of:
administering the 4-hydroxyphenylacetate 3-hydroxylase (HPAH) analogs to the compound having the general formula (I) to detect the compound having the general formula (II); and
selecting an HPAH analog with which the compound having the general formula (II) is detected.

13. A method of measuring an enzymatic activity to convert a compound having the general formula (I): 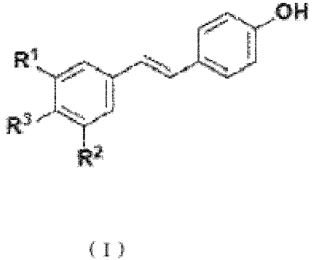 wherein
R¹ to R³ are each -H, -OH or -OR; and
R is independently selected from a C1-6 alkyl group and a glycosyl group,
or a glycoside thereof as a substrate into a compound having the general formula (II): 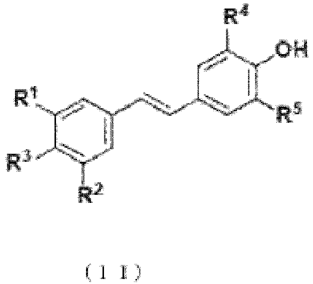 wherein
R¹ to R³ are each -H, -OH or -OR;
R is independently selected from a C1-6 alkyl group and a glycosyl group; and
one of R⁴ and R⁵ is -H and the other is -OH,
comprising the step of:
administering 4-hydroxyphenylacetate 3-hydroxylase (HPAH) to the compound having the general formula (I) to detect the compound having the general formula (II).
